# FASCICULE DE BREVET EUROPEEN

(11) **EP 1 354 574 B1**
(45) Date de publication et mention de la délivrance du brevet: **31.10.2007**
(21) Numéro de dépôt: 03006692.2
(22) Date de dépôt: 26.03.2003
(51) Int. Cl.: A61F 9/02, G02C 3/00

(54) **Masque de protection du visage utilisable notamment pour le ski ou la moto**
Gesichtsschutzmaske insbesondere zur Verwendung beim Skifahren oder Motorradfahren
Face protective goggle for use in particular for skiers or motorcyclists

(30) Priorité: 16.04.2002 FR 0205020
(43) Date de publication de la demande: 22.10.2003
(73) Titulaire: Salomon S.A., 74370 Metz-Tessy (FR)
(72) Inventeur: Schary, Philippe, 74570 Groisy (FR); Desarmaux, Pierre, 74570 Evires (FR)

(56) Documents cités:
- GB-A- 530 312
- GB-A- 921 203
- US-A- 2 758 308
- US-A- 5 706 527

## Description

L'invention concerne un masque de protection du visage utilisable notamment pour le ski ou la moto.

Notamment pour la pratique du ski il est courant d'utiliser un masque comprenant une monture, un écran de protection et une large sangle élastique qui fait le tour de la tête.

Un des problèmes de ce type de masques est la pression que la monture exerce sur le visage. Cette pression dépend de la morphologie du vissage de l'utilisateur, de la tension de la sangle élastique et de sa position sur la tête.

Notamment si la sangle élastique se trouve décalée angulairement par rapport à un plan perpendiculaire au plan défini par la monture, la pression de la monture ne s'exerce pas de façon uniforme sur le visage.

Pour résoudre ce problème on sait relier la sangle élastique à la monture non pas par une attache fixe mais par une attache pivotante qui est située vers le milieu de la monture dans le sens de la hauteur.

Ceci permet de mieux répartir la pression, toutefois il reste une concentration marquée de la pression exercée par la monture sur le visage au niveau des points d'accrochage de la sangle.

Le document US 2,758,308 montre un masque qui comprend une sangle élastique de fixation. La masque de protection comprend une monture avec deux bords latéraux, un écran porté par la monture et un lien de retenue de la monture autour de la tête d'un utilisateur qui est attaché à chacun des bords latéraux de la monture. Le lien de retenue de la monture comprend un ruban souple situé à chacune des extrémités du lien, chacun des rubans étant reliés à la monture par deux points de tirage haut et bas espacés le long d'un bord latéral selon un brin de ruban haut et un brin de ruban bas, lesdits points de tirage haut et bas permettant une modification de la longueur respective des brins haut et bas du ruban.

Un but de l'invention est d'améliorer le confort d'un masque de protection par une répartition de pression encore mieux contrôlée de la monture sur le visage.

Un autre but de l'invention est de proposer un masque pour lequel l'utilisateur peut lui-même ajuster la répartition de pression et éventuellement la mémoriser.

D'autres buts et avantages de l'invention apparaîtront au cours de la description qui va suivre.

Le masque selon l'invention est défini dans les revendications annexées.

L'invention sera mieux comprise en se référant à la description ci-dessous et aux dessins en annexe qui lui sont rattachés.
La figure 1 est une vue générale en perspective d'un masque selon un premier mode de mise en oeuvre de l'invention.
La figure 2 montre l'extrémité du ruban et la cordelette avant leur accrochage.
Les figures 3 et 4 illustrent le fonctionnement de ce mode de réalisation.
Les figures 5 et 6 sont relatives à des variantes de construction.
La figure 7 illustre une variante de mise en oeuvre de l'invention.
La figure 8 représente un autre mode de mise en oeuvre de l'invention.

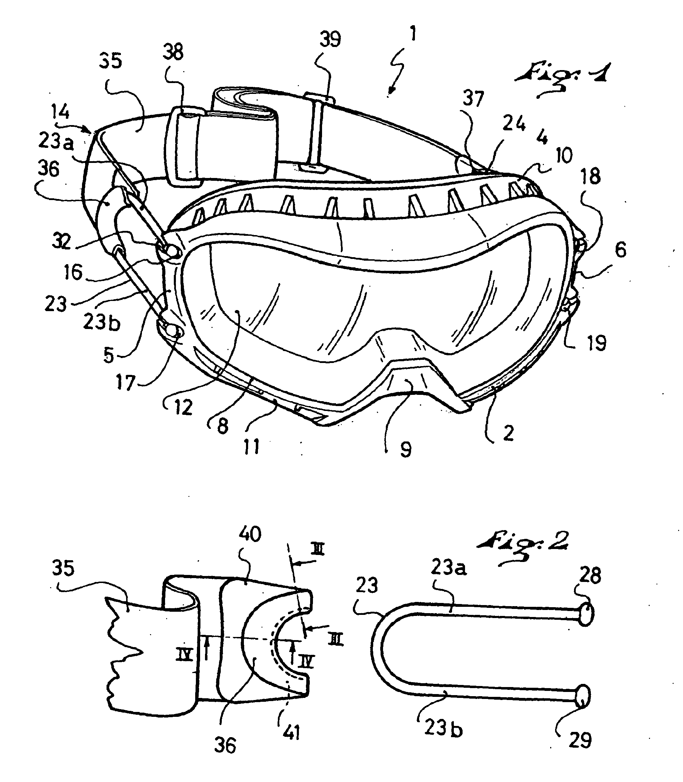

La figure 1 représente en perspective un masque 1 selon un premier mode non limitatif de mise en oeuvre de l'invention.

Le masque comprend une monture 2. La monture est de tout type et de toute forme appropriée. Par exemple elle est réalisée en matière plastique, et de façon classique elle comprend un cadre fermé de forme allongée avec un longeron supérieur 4, deux bords latéraux 5 et 6, un longeron inférieur 8 présentant un pontet médian 9 prévu pour reposer sur le nez d'un utilisateur.

La monture a de préférence au niveau de sa face arrière une lèvre 10 de contact avec le visage de l'utilisateur, éventuellement garnie d'une mousse élastiquement déformable pour améliorer le confort.

Sur le devant, la monture présente de façon optionnelle des évents d'aération 11.

De façon également classique la monture porte un écran 12 qui est retenu dans une rainure de maintien. L'écran est de tout type approprié, et il est réalisé en toute matière actuellement connue. Notamment l'écran peut être simple ou double, et être réalisé à partir d'une feuille découpée, ou par moulage. Il peut être transparent, teinté ou non teinté.

Un lien de retenue 14 est par ailleurs attaché à la monture pour maintenir le masque autour de la tête d'un utilisateur.

Selon le mode de réalisation illustré, chacun des bords latéraux 5 et 6 de la monture a des points de tirage espacés l'un de l'autre, 16 et 17 pour le bord 5, 18 et 19 pour le bord 6.

Ces points de tirage sont chacun prévus pour retenir les extrémités de deux cordelettes souples 23 et 24 qui sont situées aux extrémités du lien de retenue.

Les points de tirage sont espacés le long des bords latéraux 5 et 6 de la monture pour répartir les efforts de traction exercés par le lien sur la monture. Pour un masque de taille adulte, on a obtenu de bons résultats avec un écartement des points égal ou supérieur à 30 millimètres, et par exemple égal à 35 millimètres. Ces dimensions ne sont pas limitatives. Comme la monture est retenue par les cordelettes 23 et 24, il est préférable que les points de tirage restent au niveau des bords latéraux, et qu'ils ne soient pas situés au niveau des longerons.

De préférence les deux points de tirage d'un bord latéral sont déportés vers le longeron supérieur, et sont plus proches de ce longeron que du longeron inférieur.

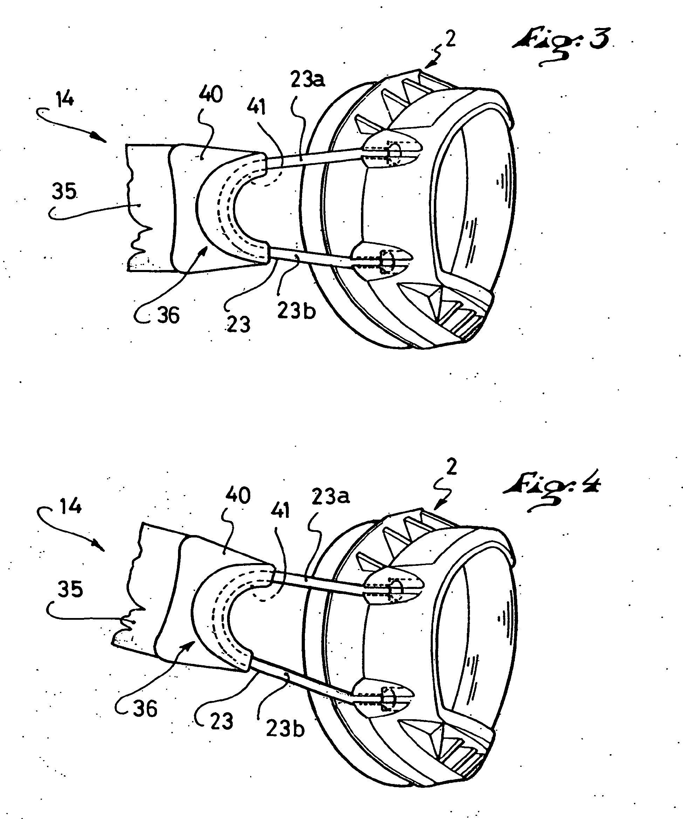

Selon le mode de réalisation illustré les cordelettes sont attachées à la monture par leurs extrémités. Par exemple tel que cela est visible en figure 2 pour la cordelette 23, les extrémités de la cordelette ont un renflement 28, 29. Ce renflement peut être réalisé de différentes façons, par exemple ils sont formés par un noeud de la cordelette elle-même, ou encore par un manchon rapporté qui est serti ou soudé à la cordelette, ou encore dans le cas où la cordelette est en matière synthétique, ils peuvent être formés par fusion et mise en forme des extrémités. D'autres techniques sont encore applicables.

Pour retenir les cordelettes 23 et 24 les points de tirage sont par exemple des logements épaulés réalisés par moulage sur les bords latéraux de la monture. La cordelette traverse le logement de part en part, son renflement d'extrémité étant retenu par l'épaulement. De préférence dans ce cas on prévoit une fente latérale par laquelle la cordelette est mise en place dans le logement. Une telle fente est repérée avec la référence 32 pour le point de tirage 16.

D'autres modes de construction peuvent aussi convenir. Par exemple on pourrait prévoir des trous traversant les bords latéraux de la monture avec éventuellement des logements pour y loger des noeuds effectués aux extrémités des cordelettes.

La cordelette est réalisée en tout matériau approprié souple. Selon le présent mode de réalisation la cordelette n'est pas extensible, ou très peu. Par exemple elle est tressée en fibres textiles synthétiques et/ou naturelles. Selon une autre possibilité la cordelette est formée par un jonc souple en matière plastique. Sa section n'est pas nécessairement constante et l'on pourrait par exemple prévoir un gainage dans sa zone de frottement avec l'élément de renvoi qui va être prochainement décrit.

La longueur de la cordelette n'est pas limitative. Il faut qu'elle soit suffisante pour déporter vers l'arrière du masque son point d'accrochage avec la pièce de renvoi.

La cordelette est reliée à un ruban élastiquement extensible 35 par des éléments de renvoi 36 et 37.

Le ruban extensible 35 est de tout type appropriée et de toute largeur appropriée constante ou non. De préférence il est réglable en longueur à l'aide de passants 38 et 39 entre lesquels le lien est détourné par des boucles.

Selon le mode de réalisation illustré chaque élément de renvoi, et en particulier l'élément 36 représenté en figure 2 a une plaquette de liaison 40 avec l'extrémité du ruban extensible 35. La plaquette est par exemple cousue, soudée ou collée à l'extrémité du ruban ou assemblée par une autre technique.

En outre l'élément a une gouttière de renvoi 41 incurvée sur environ un demi-cercle avec ses deux extrémités situées du côté de la monture. La gouttière a en section des dimensions suffisantes pour recevoir la cordelette en permettant son coulissement.

Selon le mode de réalisation illustré, la gouttière 41 forme quasiment un demi-cercle dont le diamètre est voisin de la distance entre les points de tirage. Ceci n'est pas limitatif, et l'angle d'ouverture que forme la gouttière pourrait être différent, également la gouttière pourrait avoir une autre forme qu'une forme circulaire, et la distance entre ses extrémités pourrait être différente de l'écartement entre les points de tirage de la monture. Ce qui est important c'est que la gouttière soit susceptible de recevoir la cordelette souple pour lui faire suivre une boucle de renvoi, et qu'elle permette au lien souple de coulisser. La boucle de renvoi divise la cordelette en deux brins, un brin supérieur 23a, et un brin inférieur 23b.

Les éléments de renvoi sont en tout matériau approprié, notamment en matière plastique.

Eventuellement on peut prévoir de les équiper avec une couche de mousse ou une couche textile de protection des oreilles.

Mais ceci n'est pas limitatif et les éléments de renvoi peuvent aussi être dimensionnés de manière à ne pas venir en appui sur les oreilles.

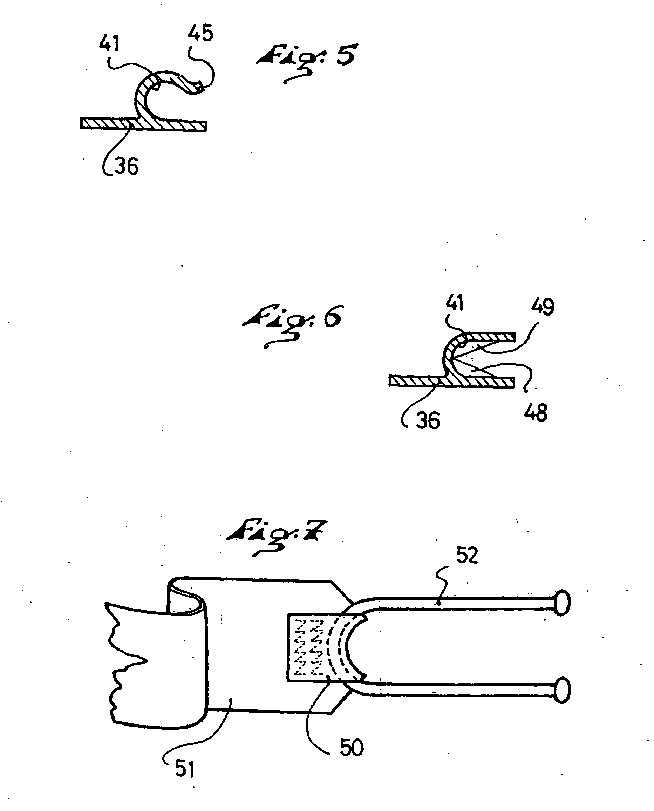

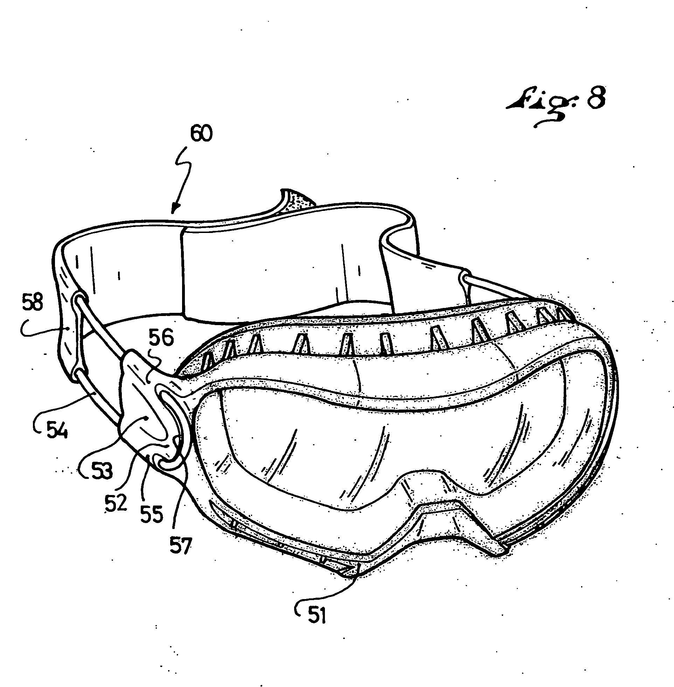

Les figures 3 et 4 illustrent le fonctionnement de ce mode de construction.

On voit qu'entre ces deux figures l'orientation du lien de retenue par rapport à la monture a été modifiée. Ce changement d'orientation résulte du coulissement de la cordelette 23 dans la gouttière 41 de l'élément de renvoi, ce qui a modifié la longueur respective des brins supérieur et inférieur 23a, 23b de la cordelette.

Ce changement d'orientation permet d'ajuster le masque à la morphologie du visage de son utilisateur, pour équilibrer la pression entre le haut ou le bas du masque ou au contraire pour modifier cette répartition. L'utilisateur a également la possibilité d'orienter le lien 14 par rapport à la monture comme il le souhaite.

Le coulissement des cordelettes dans les éléments de renvoi permet cet ajustement entre le lien et la monture du masque. La position reculée des éléments de renvoi par rapport à la monture et l'écartement des points de tirage donnent une bonne répartition de la pression de la monture contre le visage de l'utilisateur.

De plus, en cas de nécessité il est possible de retirer le masque en détachant une des cordelettes de son élément de renvoi.

Les figures 5 et 6 illustrent des variantes de construction de la gouttière.

Selon la figure 5, la gouttière 41 présente une ouverture refermée par un rebord 45. Avec le rebord, il faut franchir un point dur lors de l'introduction de la cordelette dans la gouttière ou son extraction, ce point dur étant créé par la déformation de la gouttière ou la compression de la cordelette.

Le rebord peut être présent sur toute la longueur de la gouttière ou seulement sur une partie de sa longueur. Par exemple on pourrait avoir ce rebord au niveau des deux extrémités de la gouttière. D'autres moyens peuvent aussi convenir pour obtenir un effet de point dur entre la cordelette et sa gouttière de renvoi.

Selon la figure 6 le fond de la gouttière 41 présente au moins localement des arêtes convergentes 48, 49 qui ont pour but de pincer la cordelette lorsqu'elle est attirée vers le fond de la gouttière.

Ceci permet de mémoriser un réglage de position du masque, en empêchant un coulissement de la cordelette. Le réglage est alors conservé même si on enlève le masque.

D'autres moyens pourraient aussi convenir pour immobiliser la cordelette dans la gouttière, et par exemple des picots.

En variante de ce mode de réalisation on pourrait utiliser des cordelettes extensibles avec un ruban extensible, ou encore avec un ruban inextensible.

La figure 7 montre une autre variante de réalisation. Selon cette variante, l'extrémité du ruban 51 est repliée sur le reste du ruban pour former un rabat 50 qui est assemblé au reste du ruban par exemple par couture.

Dans le mode de réalisation illustré, le rabat est moins large que le reste du ruban mais ce n'est pas limitatif.

La boucle que forme l'extrémité du ruban avec son rabat constitue un élément de renvoi dans lequel la cordelette 52 est engagée avec une possibilité de coulissement.

La figure 8 est relative à une autre variante de réalisation. Au lieu d'être attaché au lien de retenue, l'élément de renvoi fait ici partie de la monture.

Pour cela comme le montre la figure, chacun des bords latéraux de la monture 51 a un élément de renvoi 52 permettant le passage et le coulissement d'une cordelette souple 54. Avantageusement l'élément de renvoi est intégré dans la structure de la monture. Par exemple comme le montre la figure, chaque bord latéral, et en particulier le bord 53, a deux conduits 55 et 56 qui traversent le bord latéral d'arrière en avant, et qui sont reliés sur le devant par un chemin de guidage incurvé 57. En section les conduits ont des dimensions supérieures à celles du lien souple. De cette façon le lien souple 54 traverse les deux conduits 55 et 56 qui forment les points de tirage, et il suit le chemin de guidage avec une possibilité de coulissement libre.

Vers l'arrière, les deux extrémités de la cordelette sont reliées par exemple à un ruban 58 par tout moyen approprié, par exemple par couture.

Selon le mode de réalisation illustré, le ruban 60 est inextensible et il est formé de deux parties assemblées entre elles de façon détachable et réglable en longueur au moyen d'une attache connue sous la dénomination commerciale "Velcro".

Dans ce cas, de préférence, les cordelettes sont élastiquement extensibles. Ceci n'est pas limitatif, le ruban pourrait être extensible comme dans le cas précédent, et réglable en longueur à l'aide de passants. Les cordelettes pourraient aussi être inextensibles.

Selon une autre variante, les cordelettes souples pourraient être accordées l'une à l'autre de façon continue pour former le lien de retenue, c'est-à-dire que dans cette hypothèse il n'y aurait plus de ruban.

Egalement, au niveau de la monture, les éléments de renvoi pourraient être réalisés autrement.

Comme dans le cas précédent, il est possible de modifier l'orientation relative entre le lien de retenue et la monture en provoquant le coulissement des éléments de renvoi le long des cordelettes pour modifier la longueur respective de ses brins haut et bas.

Comme dans le cas précédemment décrit, on pourrait avoir des rebords pour piéger la cordelette dans le chemin de guidage et/ou des arêtes ou picots pour l'immobiliser.

Naturellement la présente description n'est donnée qu'à titre indicatif, et l'on pourrait adopter d'autres mises en oeuvre de l'invention sans pour autant sortir du cadre de celle-ci.

Enfin l'invention n'est pas limitée à un masque pour le ski ou la moto, et peut être appliquée à d'autres domaines, comme par exemple les masques de plongée.

## Revendications

1. Masque de protection pour le ski ou la moto, comprenant une monture (2, 51) avec deux bords latéraux (5, 6, 53), un écran (12) porté par la monture et un lien de retenue (14, 60) de la monture autour de la tête d'un utilisateur qui est attaché à chacun des bords latéraux de la monture, le lien de retenue (14, 60) de la monture comprenant une cordelette souple (23, 24, 54) située à chacune des extrémités du lien, chacune des cordelettes étant reliée à la monture par deux points de tirage haut et bas (16, 17, 18, 19, 55, 56) espacés le long d'un bord latéral selon un brin de cordelette haut (23a) et un brin de cordelette bas (23b), un élément de renvoi (36, 57) coulissant le long de la cordelette pour permettre une modification de la longueur respective des brins haut et bas de la cordelette, le lien de retenue (14) comprenant un ruban (35), et les éléments de renvoi (36, 37) étant situés aux extrémités du ruban.

2. Masque selon la revendication 1, **caractérisé par le fait que** les points d'attache sont distants de 30 à 40 millimètres le long des bords latéraux (5, 6) de la monture.

3. Masque selon la revendication **1, caractérisé par le fait que** l'élément de renvoi (36) comprend une gouttière (41) incurvée dans laquelle est engagée une cordelette (23).

4. Masque selon la revendication 1, **caractérisé par le fait que** les cordelettes sont attachées par des renflements (28, 29) de leurs extrémités.

5. Masque selon la revendication 1, **caractérisé par le fait que** les renflements (28, 29) sont logés dans des logements épaulés réalisés dans les bords latéraux (5, 6) de la monture.

6. Masque selon la revendication 1, **caractérisé par le fait que** le lien comporte un ruban (35) dont les extrémités sont repliées selon un rabat (50) pour former une boucle dans laquelle est engagée la cordelette (52) avec une possibilité de coulissement.

7. Masque selon la revendication 1, **caractérisé par le fait que** chacun des éléments de renvoi est un chemin de guidage (57) formé dans les bords latéraux (53) de la monture.

8. Masque selon la revendication 7, **caractérisé par le fait que** les extrémités des cordelettes sont attachées aux extrémités d'un ruban (58).

9. Masque selon la revendication 7, **caractérisé par le fait que** les cordelettes sont reliées entre elles de façon continue.

## Claims

1. Protective goggles for skiing or motorcycling, comprising a frame (2, 51) with two lateral edges (5, 6, 53), a shield (12) borne by the frame and a tie (14, 60) for retaining the frame on a user's head which is attached to each of the lateral edges of the frame, the tie (14, 60) for retaining the frame comprising a flexible cord (23, 24, 54) located at each of the tie ends, each of the cords being connected to the frame by two upper and lower pull points (16, 17, 18, 19, 55, 56) spaced apart along a lateral edge at an upper cord point (23a) and a lower cord point (23b), a return element (36, 57) sliding along the cord to allow modifying the respective length of the upper and lower cord points, the retaining tie (14) comprising a band (35), and the return elements (36, 37) being located at the ends of the band.

2. Goggles according to claim 1, **characterized in that** the pull points are separated by 30-40 millimeters along the lateral edges (5, 6) of the frame.

3. Goggles according to claim 1, **characterized in that** the return element (36) comprises an inward curved channel (41) in which a cord (23) is engaged.

4. Goggles according to claim 1, **characterized in that** the cords are attached by bulged ends (28, 29).

5. Goggles according to claim 1, **characterized in that** the bulges (28, 29) are housed in shouldered housings made in the lateral edges (5, 6) of the frame.

6. Goggles according to claim 1, **characterized in that** the tie comprises a band (35) whose ends are folded along a flap (50) to form a loop in which the cord (52) is engaged with a possibility for sliding.

7. Goggles according to claim 1, **characterized in that** each of the return elements is a guiding track (57) formed in the lateral edges (53) of the frame.

8. Goggles according to claim 7, **characterized in that** the ends of the cords are attached to the ends of a band (58).

9. Goggles according to claim 7, **characterized in that** the cords are connected together in a continuous manner.

## Patentansprüche

1. Schutzmaske für das Ski- oder Motorradfahren mit einem Gestell (2, 51) mit zwei Seitenrändern (5, 6, 53), einem durch das Gestell getragenen Schirm (12) und einer Verbindung (14, 60), um das Gestell um den Kopf eines Benutzers zu halten, welche an jedem der Seitenränder des Gestells befestigt ist, wobei die Halteverbindung (14, 60) des Gestells eine flexible Schnur (23,24, 54) umfasst, die sich an jedem der Enden der Verbindung befindet, wobei jede Schnur über zwei Zugpunkte, einen oberen und einen unteren (16, 17, 18, 19, 55, 56), die entlang eines Seitenrandes beabstandet sind, mit dem Gestell verbunden ist, gemäß einem oberen Schnurstrang (23a) und einem unteren Schnurstrang (23b), wobei ein Umkehrelement (36, 57) entlang der Schnur gleitet, um eine Veränderung der jeweiligen Länge des oberen und unteren Strangs der Schnur zu erlauben, wobei die Halteverbindung (14) ein Band (35) umfasst und die Umkehrelemente (36, 37) sich an den Enden des Bandes befinden.

2. Maske nach Anspruch 1, **gekennzeichnet durch** die Tatsache, dass die Befestigungspunkte entlang der Seitenränder (5, 6) des Gestells um 30 bis 40 Millimeter beabstandet sind.

3. Maske nach Anspruch 1, **gekennzeichnet durch** die Tatsache, dass das Umkehrelement (36) eine gekrümmte Rinne (41) umfasst, in welcher eine Schnur (23) eingesetzt ist.

4. Maske nach Anspruch 1, **gekennzeichnet durch** die Tatsache, dass die Schnüre über Verdickungen (28, 29) ihrer Enden befestigt sind.

5. Maske nach Anspruch 1, **gekennzeichnet durch** die Tatsache, dass die Verdickungen (28, 29) in Schultersitzen untergebracht sind, die in den Seitenrändern (5, 6) des Gestells ausgebildet sind.

6. Maske nach Anspruch 1, **gekennzeichnet durch** die Tatsache, dass die Verbindung ein Band (35) aufweist, dessen Enden gemäß einem umgeschlagenen Ende (50) umgeschlagen sind, um eine Schlaufe zu bilden, in welcher die Schnur (52) gleitfähig eingesetzt ist.

7. Maske nach Anspruch 1, **gekennzeichnet durch** die Tatsache, dass jedes der Umkehrelemente ein Führungsabschnitt (57) ist, der in den Seitenrändern (53) des Gestells ausgebildet ist.

8. Maske nach Anspruch 7, **gekennzeichnet durch** die Tatsache, dass die Enden der Schnüre an den Enden eines Bandes (58) befestigt sind.

9. Maske nach Anspruch 7, **gekennzeichnet durch** die Tatsache, dass die Schnüre kontinuierlich miteinander verbunden sind.
